(19) Europäisches Patentamt
European Patent Office
Office européen des brevets

(11) EP 1 284 134 B1

(12) **EUROPEAN PATENT SPECIFICATION**

(45) Date of publication and mention of the grant of the patent:
**28.03.2007 Bulletin 2007/13**

(21) Application number: **01932233.8**

(22) Date of filing: **23.05.2001**

(51) Int Cl.:
***A61K 8/49*** *(2006.01)* ***A61K 8/97*** *(2006.01)*
***A61K 31/4406*** *(2006.01)* ***A61K 36/54*** *(2006.01)*
***A61K 36/73*** *(2006.01)* ***A61Q 19/00*** *(2006.01)*
***A61P 17/10*** *(2006.01)*

(86) International application number:
**PCT/JP2001/004336**

(87) International publication number:
**WO 2001/089471 (29.11.2001 Gazette 2001/48)**

(54) **EXTERNAL SKIN PREPARATIONS FOR SUPPRESSING SEBUM SECRETION**

ÄUSSERLICH ANZUWENDENDE ZUBEREITUNGEN FÜR DIE HAUT ZUR UNTERDRÜCKUNG DER SEBUM-SEKRETION

PREPARATIONS POUR LA PEAU A USAGE EXTERNE PERMETTANT DE SUPPRIMER LA SECRETION DE SEBUM

(84) Designated Contracting States:
**DE FR IT**

(30) Priority: **26.05.2000 JP 2000197309**
**21.05.2001 JP 2001151391**

(43) Date of publication of application:
**19.02.2003 Bulletin 2003/08**

(73) Proprietor: **SHISEIDO COMPANY, LTD.**
**Chuo-ku,**
**Tokyo 104-8010 (JP)**

(72) Inventors:
- **INOMATA, Shinji,**
**Shiseido Res. Ctr.(Shin-Yokohama)**
**Yokohama-shi, Kanagawa 224-8558 (JP)**
- **KOBAYASHI, Koji,**
**Shiseido Res. Ctr.(Shin-Yokohama)**
**Yokohama-shi, Kanagawa 224-8558 (JP)**

(74) Representative: **Santarelli**
**14, avenue de la Grande Armée**
**75017 Paris (FR)**

(56) References cited:
**EP-A- 0 606 046** **EP-A- 0 985 409**
**JP-A- 8 048 625** **JP-A- 59 164 712**
**JP-A- 63 215 612**

- **DATABASE WPI Derwent Publications Ltd., London, GB; AN 2000-579204 XP002316465 & WO 00/51562 A (SHISEIDO CO LTD) 8 September 2000 (2000-09-08)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 284 134 B1

## Description

Field of Invention

The present invention relates to drugs for external application to the skin for inhibiting sebum secretion. More specifically, the present invention relates to the use of an active ingredient having an excellent antagonistic effect against matrix metalloproteinase activity for the production of drugs for inhibiting sebum secretion.

One of the pharmaceutical properties required, for a long time, for the prevention and/or improvement of acne and oily skins etc. is the effect of inhibiting sebum secretion. As conventional drugs for inhibiting sebum, there have been reported plant extracts, vitamins, female hormones, etc. Except for female hormones and some of the vitamin derivatives, however, their effect was weak and was not satisfactory. Since sebum secretion is controlled by male hormones, there have been cases in which estrogenic hormones, specifically estradiol, estrone, etc., that have an antagonistic effect against them were blended as a drug for inhibiting sebum secretion (Shin-Keshohin Gaku (New Cosmetology), page 167, Nanzando (1993)). However, they have a risk of side effects, and therefore have major limitations on the blending, and thus they are not satisfactory when they are put into practical use.

The possible mechanism of drugs for inhibiting sebum secretion is two fold: the effect of suppressing the growth of sebaceous gland cells and the effect of inhibiting the lipid synthesis of sebaceous gland cells. Since the former includes the above female hormones having potential effects, there is a need for drugs, for inhibiting sebum secretion, that exhibit equivalent effects without being accompanied by side effects (without hormone actions). However, no such substances have been found so far.

Disclosure of the Invention

The present inventors have performed an in-depth investigation on the mechanism responsible for the growth of sebaceous gland cells and sebum secretion, and have found that matrix metalloproteinase is enhanced in the sebaceous gland cells which have come to secrete sebum in excess and, specifically, gelatinase, belonging to the matrix metalloproteinases, is enhanced. When substances inhibiting these enzymes were externally applied, the effects of lowering enzyme activity and suppressing the growth of sebaceous gland cells, and furthermore, the desired effect of inhibiting sebum secretion were found, based on which the present invention was completed. Specific experimental data are shown in Example 1 below.

Thus, the present invention relates to the use of a matrix metalloproteinase inhibitor as an active ingredient for the production of drugs for external application for inhibiting sebum secretion.

Brief Explanation of the Drawings

In Fig. 1, A shows the result of observation, with in situ zymography, of gelatinase in the sebaceous gland area of a human forehead. In the figure, a broken line represents the contour of the sebaceous gland area, and the white area indicated by the arrow represents gelatin degradation activity.

B shows the result of observation, with in situ zymography, of gelatinase in the sebaceous gland area of a human scalp. In the figure, a broken line represents the contour of the sebaceous gland area, and the white area indicated by the arrow indicates gelatin degradation activity.

In Fig. 2, A shows a hematoxylin-eosin stain of the skin of a mouse that was not irradiated with UVB.

B shows a hematoxylin-eosin stain of the skin of a mouse that was repeatedly irradiated with UVB for 10 weeks.

In Fig. 3, A shows the result of observation, with in situ zymography, of gelatinase behavior in the sebaceous gland area of the skin not irradiated with UVB. The arrow indicates the sebaceous gland area.

B shows the result of observation, with in situ zymography, of gelatinase behavior in the sebaceous gland area of the skin irradiated with UVB for 10 weeks. The arrow indicates the sebaceous gland area.

C shows the result of observation, with in situ zymography, of gelatinase behavior in the sebaceous gland area of the skin treated with a 10 week irradiation of UVB + EDTA (100 mM). The arrow indicates the sebaceous gland area.

In Fig. 4, A shows the result of observation, with in situ zymography, of the sebaceous gland area of the skin to which an active ingredient A, a matrix metalloproteinase inhibitor, was transdermally applied, and a broken line indicates the result of the epidermal area and the sebaceous gland organ area.

B shows the result of in situ zymography of the skin to which a solvent was only applied, and a broken line indicates the result of the epidermal area and the sebaceous gland organ area.

Embodiment for Carrying Out the Invention

In accordance with the present invention, an active ingredient of the drug for inhibiting sebum secretion may

be any substance that inhibits matrix metalloproteinase, and there can be mentioned for example N-hydroxy-2(R)-[[4-methoxyphenyl)sulfonyl](3-picolyl)amino]-3-methylbutanamide (termed as active ingredient A) belonging to the hydroxamic acid derivatives, or a salt thereof, for example a hydrochloride. The structure of the hydrochloride of active ingredient A is represented by the following formula:

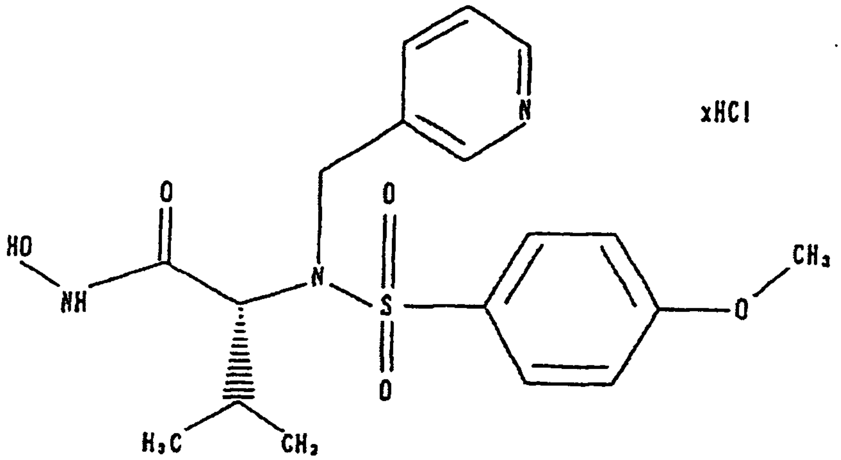

Furthermore, as substances that inhibit matrix metalloproteinase, there can be mentioned doxycycline having a tetracycline backbone, and curcumine, as a natural compound, for which an excellent gelatinase inhibiting effect was found, and plant extracts (turmeric extract) containing it, and the following plant extracts for which the MMPs inhibiting effect has been confirmed:

Thymus serpyllum L., Valeriana fauriei Briquet or its related plants (Valerianaceae), Diospyros kaki Thunberg (Ebenaceae), Astragalus sinicus Linne (Leguminosae), Crataegus cuneata Siebold et Zuccarini (Rosaceae), Paeonia suffruticosa Andrews (Poeonia montan Sims) (Paeoniaceae), Theasinensis Linne var. assamica Pierre (Theaceae), Eucalyptus globulus Labillardiere or its related plants (Myrtaceae), Potentilla tormentilla Schrk (Rosaceae), Tilia cordata Mill., Tillia platyphyllus Scop., Tilia europaea Linne (Tiliaceae), Betulaalba Linne (Betulaceae), Origanum majorana L., Uncaria gambir Roxburgh (Rubiaceae), Juglans regia Linne var. sinensis De Candolle or its related plants (Juglandaceae), Sophora flavescens Aiton (Leguminosae), Sanguisorba officinalis Linne (Rosaceae), Hypericum perforatum Linne or Hypericum erectum Thunberg (Guttiferae), Thea sinensis Linne (Theaceae), Symplocos racemosa, Cyperus rotundus, Cyperus scariosus, Gaultheria fragrantissima, Acacia fornensia, Terminalia chebula, Ficus bengalensis, Cassiafistula Linn, Lyonia ovalifolia, Calophyllum inophyllum, Ficus religiosa, Persea americana Mill, Garcinia mangostan, Cocos nucifera L, Blumea balsamifera (L) DC., Woodfordia floribunda Salisb., and Cinnamomum cassin.

The constitution of the present invention will be explained below.

The present inventors have found, for the first time, that drugs for external application to the skin containing a matrix metalloproteinase antagonist for use in the present invention exhibit the effect of inhibiting the sebum secretion of the skin.

In accordance with the present invention, matrix metalloproteinases include, for example, gelatinase. Thus, the metalloproteinase inhibitor of the present invention is a substance that inhibits the above-mentioned matrix metalloproteinases.

Preferably, the drugs for external application containing a matrix metalloproteinase antagonist for use in the present invention are used as cosmetics for inhibiting sebum secretion, and the amount blended of the matrix metalloproteinase antagonist is 0.0001-20% by weight in tems of dry weight, preferably 0.0001-10.0% by weight relative to the total amount of the drug. When the amount is less than 0.0001% by weight, the effect mentioned in the present invention cannot be fully exhibited, and when it exceeds 20.0% by weight, it is difficult to formulate into drugs and thus is undesirable. Furthermore, the blending of the amount over 10.0% by weight does not result in significant increasess in efficacy.

In addition to the above-mentioned essential ingredients, the matrix metalloproteinase antagonist-blended drugs for external application of the present invention may be blended, as appropriate, with ingredients used for drugs for external application such as common cosmetics and pharmaceutical drugs, including for example whitening agents, moisturizers, antioxidants, oily components, UV absorbers, surfactants, thickening agent, alcohols, powder components, coloring agents, aqueous components, water, various skin nutrients, as needed.

In addition, there may be blended, as appropriate, sequestering agents such as edetate disodium, edetate trisodium, sodium citrate, sodium polyphosphate, sodium metaphosphate, and gluconic acid, caffeine, tannin, verapamil,

tranexamic acid and derivatives thereof, licorice extracts, glabridin, hot water extracts of padauk fruit, various crude drugs, tocopherol acetate, drugs such as glycyrrhetic acid and derivatives thereof or salts thereof, vitamin C, ascorbic acid magnesium phosphate, ascorbic acid glucoside, arbutin, other whitening agents such as kojic acid, sugars such as glucose, fructose, mannose, sucrose and trehalose, vitamin A such as retinoic acid, retinol, retinol acetate, and retinol palmitate.

Drugs for external application to the skin containing the matrix metalloproteinase antagonist of the present invention may be any substances that are conventionally used in drugs for external application to the skin such as ointments, creams, emulsions, lotions, packs, and baths, and the dosage forms are not specifically limited.

Examples

The present invention will now be explained in more details with examples. It should be noted that the present invention is not limited by these examples. The amount blended is expressed in % by weight.

Formulation examples of active ingredient A in various dosage forms by the present invention are explained as Examples.

Example 1. Cream

(Formulation)

| | |
|---|---|
| Stearic acid | 5.0 % by weight |
| Stearyl alcohol | 4.0 |
| Isopropyl myristate | 18.0 |
| Glycerin monostearate | 3.0 |
| Propylene glycol | 10.0 |
| Hydrochloride of active ingredient A | 1.0 |
| Caustic potash | 0.2 |
| Sodium bisulfite | 0.01 |
| Preservative | Proper amount |
| Perfume | Proper amount |
| Ion exchanged water | Balance |

(Method of preparation)

To ion exchanged water, propylene glycol, active ingredient A and caustic potash are added and dissolved, and then heated and maintained at 70°C (aqueous phase). The other ingredients are mixed, melted under heating and maintained at 70°C (oily phase). The oily phase is gradually added to the aqueous phase, and after the total amount has been added, the temperature is maintained for some time in order to allow a reaction to occur. It is then homogeneously emulsified by a homomixer, and cooled to 30°C under sufficient stirring.

Example 2. Cream

(Formulation)

| | |
|---|---|
| Stearic acid | 2.0% by weight |
| Stearyl alcohol | 7.0 |
| Hydrogenated lanolin | 2.0 |
| Squalane | 5.0 |
| 2-octyldodecyl alcohol | 6.0 |
| Polyoxyethylene (25 mole) cetylalcohol ether | 3.0 |
| Glycerin monostearate | 2.0 |

(continued)

| | |
|---|---|
| Propylene glycol | 5.0 |
| Extract of tormentilla (dry weight) | 0.05 |
| Sodium bisulfite | 0.03 |
| Ethyl paraben | 0.3 |
| Flavor | Proper amount |
| Ion exchanged water | Balance |

(Method of preparation)

To ion exchanged water, propylene glycol is added, and heated and maintained at 70°C (aqueous phase). The other ingredients are mixed, melted under heating and maintained at 70°C (oily phase). The oily phase is added to the aqueous phase to pre-emulsify, and after homogeneously emulsified by a homomixer, it is cooled to 30°C under sufficient stirring.

Example 3. Cream

(Formulation)

| | |
|---|---|
| Solid paraffin | 5.0 % by weight |
| Beeswax | 10.0 |
| Vaseline | 15.0 |
| Liquid paraffin | 41.0 |
| Glycerin monostearate | 2.0 |
| Polyoxyethylene (20 mole) sorbitan monolaurate | 2.0 |
| Soap powder | 0.1 |
| Borax | 0.2 |
| Curcumine extract (dry weight) | 0.01 |
| Sodium bisulfite | 0.03 |
| Ethyl paraben | 0.3 |
| Perfume | Proper amount |
| Ion exchanged water | Balance |

(Method of preparation)

To ion exchanged water, soap powder and borax are added, dissolved under heating, and maintained at 70°C (aqueous phase). The other ingredients are mixed, melted under heating, and maintained at 70°C (oily phase). The oily phase is gradually added to the aqueous phase to allow a reaction to occur. After the reaction is complete, it is homogeneously emulsified by a homomixer, and after emulsification it is cooled to 30°C under sufficient stirring.

Example 4. Emulsion

(Formulation)

| | |
|---|---|
| Stearic acid | 2.5 % by weight |
| Cetyl alcohol | 1.5 |
| Vaseline | 5.0 |
| Liquid paraffin | 10.0 |
| Polyoxyethylene (10 mole) monooleate | 2.0 |
| Polyethylene glycol 1500 | 3.0 |

(continued)

| | |
|---|---|
| Triethanolamine | 1.0 |
| Carboxyvinyl polymer (Trade name: Carbopol 941, B.F. Goodrich Chemical company) | 0.05 |
| Avocado extract (dry weight) | 0.01 |
| Sodium bisulfite | 0.01 |
| Ethyl paraben | 0.3 |
| Flavor | Proper amount |
| Ion exchanged water | Balance |

(Method of preparation)

To a small amount of ion exchanged water, carboxyvinyl polymer is dissolved (phase A). To the rest of the ion exchanged water, polyethylene glycol 1500 and triethanolamine are added, dissolved under heating, and maintained at 70°C (aqueous phase). The other ingredients are mixed, melted under heating, and maintained at 70°C (oily phase). The oily phase is added to the aqueous phase to pre-emulsify, to which phase A is added and homogeneously emulsified by a homomixer. After emulsification, it is cooled to 30°C under sufficient stirring.

Example 5. Emulsion

(Formulation)

| | |
|---|---|
| Microcrystalline wax | 1.0 % by weight |
| Beexwax | 2.0 |
| Lanoline | 20.0 |
| Liquid paraffin | 10.0 |
| Squalane | 5.0 |
| Sorbitan sesquioleate | 4.0 |
| Polyoxyethylene (20 mole) sorbitan monooleate | 1.0 |
| Propylene glycol | 7.0 |
| Avocado extract (dry weight) | 10.0 |
| Sodium bisulfite | 0.01 |
| Ethyl paraben | 0.3 |
| Perfume | Proper amount |
| Ion exchanged water | Balance |

(Method of preparation)

To ion exchanged water, propylene glycol is added, and heated and maintained at 70°C (aqueous phase). The other ingredients are mixed, melted under heating, and maintained at 70°C (oily phase). While the oily phase is stirred, the aqueous phase is gradually added thereto, and homogeneously emulsified by a homomixer. After emulsification, it is cooled to 30°C under sufficient stirring.

Example 6. Jelly

(Formulation)

| | |
|---|---|
| 95% ethyl alcohol | 10.0 % by weight |
| Dipropylene glycol | 15.0 |
| Polyoxyethylene (50 mole) oleylalcohol ether | 2.0 |
| Carboxyvinyl polymer (Trade name: Carbopol 940, B.F. Goodrich Chemical company) | 1.0 |

(continued)

| | |
|---|---|
| Caustic soda | 0.15 |
| L-arginine | 0.1 |
| Mangostin extract (dry weight) | 7.0 |
| Sodium 2-hydroxy-4-methoxybenzophenone sulfonate | 0.05 |
| Ethylenediaminetetraacetic acid trisodium dihydrate | 0.05 |
| Methyl paraben | 0.2 |
| Perfume | Proper amount |
| Ion exchanged water | Balance |

(Method of preparation)

To ion exchanged water, Carbopol 940 is homogeneously dissolved, while the hydrochloride of active ingredient A and polyoxyethylene (50 mole) oleylalcohol ether are dissolved and added to the aqueous phase. Then, the other ingredients are added, and caustic soda and L-arginine are added to neutralize it and to increase viscosity.

Example 7. Beauty lotion

(Formulation)

| | |
|---|---|
| (Phase A) | |
| Ethyl alcohol (95%) | 10.0 % by weight |
| Polyoxyethylene (20 mole) octyldodecanol | 1.0 |
| Panthothenilethyl ether | 0.1 |
| Coconut extract (dry weight) | 1.5 |
| Methyl paraben | 0.15 |
| (Phase B) | |
| Potassium hydroxide | 0.1 |
| (Phase C) | |
| Glycerin | 5.0 |
| Dipropylene glycol | 10.0 |
| Sodium bisulfite | 0.03 |
| Carboxyvinyl polymer (Trade name:Carbopol 940, B.F. Goodrich Chemical company) | 0.2 |
| Purified water | Balance |

(Method of preparation)

Phase A and phase B are separately dissolved, and phase A is added to phase C. Then phase B is added thereto and filling is performed.

Example 8. Pack

(Formulation)

| | |
|---|---|
| (Phase A) | |
| Dipropylene glycol | 5.0 % by weight |
| Polyoxyethylene (60 mole) hydrogenated castor oil | 5.0 |

(continued)

| | |
|---|---|
| (Phase B) | |
| Extract of Blumea balsamifera (dry weight) | 0.01 |
| Olive oil | 5.0 |
| Tocopherol acetate | 0.2 |
| Ethyl parabene | 0.2 |
| Perfume | 0.2 |
| (Phase C) | |
| Sodium bisulfite | 0.03 |
| Polyvinyl alcohol (degree of saponification 90, degree of polymerization 2,000) | 13.0 |
| Ethanol | 7.0 |
| Purified water | Balance |

(Method of preparation)

Phase A, phase B and phase C are separately, homogeneously dissolved, and phase A is added to phase B to solubilize. Then this is added to phase C and filling is performed.

Example 9. Solid foundation

(Formulation)

| | |
|---|---|
| Talc | 43.1 % by weight |
| Kaolin | 15.0 |
| Cericite | 10.0 |
| Zinc flower | 7.0 |
| Titanium dioxide | 3.8 |
| Yellow iron oxide | 2.9 |
| Black iron oxide | 0.2 |
| Squalane | 8.0 |
| Isostearic acid | 4.0 |
| Monooleic acid POE sorbitan | 3.0 |
| Isocetyl Octoate | 2.0 |
| Extract of Cinnamomum eassin (dry weight) | 1.0 |
| Preservative | Proper amount |
| Perfume | Proper amount |

(Method of preparation)

Powder components of talc and black iron oxide are mixed well in the blender, and then oily components of squalane to isocetyl octaoate, the hydrochloride of active ingredient A, a preservative and a flavor are added thereto, and after blending well, it is filled into a container and molded.

Example 10. Emulsified foundation (cream type)

(Formulation)

| | |
|---|---|
| (Powder portion) | |
| Titanium dioxide | 10.3 % by weight |

(continued)

| | |
|---|---|
| (Powder portion) | |
| Cericite | 5.4 |
| Kaolin | 3.0 |
| Yellow iron oxide | 0.8 |
| Red iron oxide | 0.3 |
| Black iron oxide | 0.2 |
| (oily phase) | |
| Decamethylcyclopentasiloxane | 11.5 |
| Liquid paraffin | 4.5 |
| Polyoxyethylene modified Dimethylpolysiloxane | 4.0 |
| (aqueous phase) | |
| Purified water | 50.0 |
| 1,3-butyleneglycol | 4.5 |
| Tormentilla extract (dry weight) | 1.5 |
| Sorbitan sesquioleate | 3.0 |
| Preservative | Proper amount |
| Perfume | Proper amount |

(Method of preparation)

After the aqueous phase is stirred under heating, a fully mixed and ground powder portion is added thereto and treated by a homomixer. After the heated and mixed oily phase is further added and treated by a homomixer, a flavor is added under stirring, and then cooled to room temperature.

Experiment 1. Detection of gelatinase activity in the sebaceous gland

(1) The detection of gelatinase activity in the human sebaceous gland area

By the in situ zymography method using the FIZ-GN film by Fuji Photo Film, gelatin degradation activity at the sebaceous gland area was degraded (S. Inomata et al., J. Invest. Dermatol., 114(4):822 (2000); H. Nakamura et al., Cancer Res., Vol. 59, 467 (1999)). By staining gelatin with the Ponceau solution, the gelatin digested area (the area in which the gelatinase activity is present) was rendered white for detection. Fig. 1A shows the result of an in situ zymography of the sebaceous gland tissue in the forehead area of the normal skin, and Fig. 1B shows the result of an in situ zymography of each sebaceous gland tissue present in the scalp area. It can be seen that little gelatinase activity is detected in the sebaceous gland in the scalp whereas in the forehead area in which sebaceous glands are well developed, gelatinase activity is enhanced.

(2) The development of the in vivo skin sebaceous gland area and the behavior of gelatinase activity

It is known that when UVB (50 mJ/cm$^2$ - 200 mJ/cm$^2$; 50 mJ/cm$^2$ at the start of irradiation, the amount of irradiation is increased stepwise depending on the number of irradiations) below the erythema formation level is repeatedly irradiated to hairless mice, the sebaceous gland becomes developed and the amount of sebum increases (R.H. Lesnik et al., Arch Dermatol., Vol. 284, 106 (1992)). Fig. 2A and Fig. 2B represent the H&E stains of the UVB-non-irradiated tissue and the UVB-irradiated tissue. It is observed that UVB irradiation results in the thickening of the epidermis as well as the development of the sebaceous gland.

Fig. 3A, Fig. 3B and Fig. 3C show the result of gelatinase activity in the periphery of the sebaceous gland area examined by an in situ zymography. In the UVB non-irradiated epidermal area, no gelatinase activity is observed, and only a slight activity is observed in the sebaceous gland area (Fig. 3A). In contrast, a very potential gelatinase activity is evident in the UVB irradiated and thereby developed epidermal area and sebaceous gland areas (Fig. 3B) (S. Inomata et al., Jpn. J. Dermatol., Vol. 111(3), 532 (2000)). It is possible that the gelatin degradation activity detected in an in situ zymography may result from proteinases other than gelatinase (MMPs), but the addition of a MMPa inhibitor (EDTA) in the in situ zymography reaction process in vitro inhibited the gelatin degradation at the epidermal area and the sebaceous gland area, as shown in Fig. 3C, it is obvious that it results from a gelatinase belonging to the MMPs (S. Inomata et al., Jpn. J. Dermatol., Vol. 111(3), 532 (2000)).

(3) Effect of the transdermal application of an MMPs inhibitor on the sebaceous gland

The effect of the transdermal application of an MMPs inhibitor on the sebaceous gland development and the gelatinase activity present in the sebaceous gland is shown for active substance A as an example (Fig. 4A and Fig. 4B). It can be seen that the application of an MMPs inhibitor suppresses the development of the sebaceous gland area that otherwise develops by UVB stimulation, and the gelatinase activity is also inhibited.

Experiment 2. Experiment on the inhibitory effect of various MMPs inhibitors on sebum secretion

(1) Experimental condition

UV is generally known to activate the sebaceous gland cells on the skin and thereby to enhance the amount of sebum secretion, which has been reported on a model using hairless mice (R.H. Lesnik et al., Arch Dermatol., Vol. 284, 106 (1992)). In an experiment, Bissett et al. (Phytochem. Phytobiol, Vol. 46, 3, 367 (1987)) applied the method of preparing a light-aged skin, and induced the activation of sebaceous gland cells and enhancement in the amount of sebum by irradiating the dorsal area of the hairless mice repeatedly three times per week for 5-8 weeks by controlling the amount of UVB irradiation to under the erhythema formation level.

In the course of the UV irradiation, 100 μl of 1% of the test substance was applied three times per week immediately after each UV irradiation. As the control, a group in which the solvent for the test substance was applied in a similar protocol was set up, and the effect of inhibiting sebum was evaluated by comparing the amount of sebum between the two. The measurement of sebum amount was performed using a sebumeter (COURAGE KHAZAKA company). From the measured values, the ratio (%) of sebum inhibition was calculated based on the following criteria:

$$\left[1 - \frac{\text{Average of sebum in test substance group}}{\text{Average of sebum in solvent group}}\right] \times 100$$

As can be seen from Table 1, an excellent effect of inhibiting sebum secretion was observed for matrix metalloproteinase inhibitors, which indicates that it is a very favorable formulation method to blend a matrix metalloproteinase inhibitor as an active ingredient of the drug for inhibiting sebum secretion.

Table 1

| Effect of inhibiting sebum secretion | |
|---|---|
| Test substance | Ratio of sebum inhibition (%) |
| Hydrochloride of active ingredient A | 79 |
| Extract of Potentilla tormentilla S. | 99 |
| Curcumine | 77 |
| Extract of Persea americana Mill. | 70 |
| Extract of Garcincia mangostana L. | 73 |
| Extract of Cocos nucifera L. | 100 |
| Extract of Blumea balsamifera | 80 |
| Extract of Woodfordia floribunda Salisb. | 51 |
| Extract of Cinamomum cassia B1. | 93 |
| <Positive control> Estradiol 0.6 mg/kg oral administration | 65 |

Industrial Applicability

From the foregoing, the matrix metalloproteinase inhibitor-blended drugs for external application to the skin of the present invention exhibit the effect of inhibiting sebum secretion, and act favorably in preventing and/or improving acne, oily skins, keratotic plug inhibition, and pore reduction.

## Claims

1. The use of a matrix metalloproteinase inhibitor for the production of a composition for external application to the skin for inhibiting sebum secretion.

2. The use according to claim 1 wherein the matrix metalloproteinase is a proteinase belonging to the gelatinase group.

3. The use according to claim 1 wherein said matrix metalloproteinase inhibitor is selected from the group consisting of active substance A represented by the following formula:

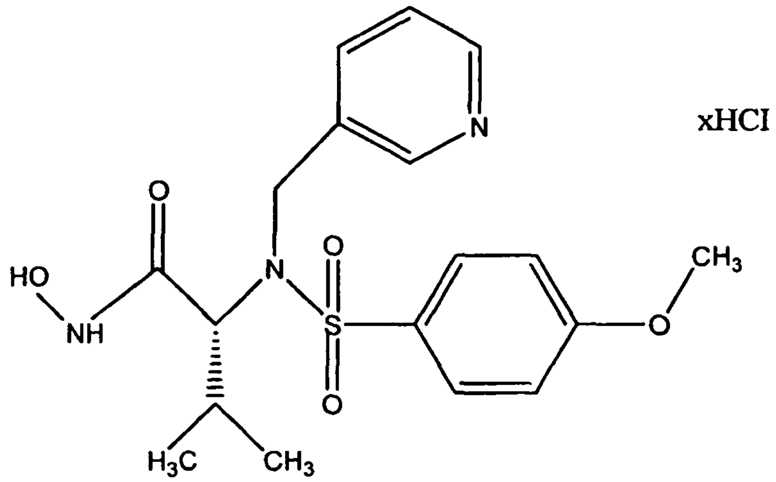

, an extract of Patentilla tormentilla S., Curcumine, an extract of Persea americana Mill., an extract of Garcinia mangostana L., an extract of Cocos nucifera L., an extract of Blumea balsamifera (L) DC., and an extract of Cinnamomum cassia B1.

## Patentansprüche

1. Verwendung eines Matrixmetalloproteinaseinhibitors zur Herstellung einer Zusammensetzung zur äußerlichen Anwendung auf die Haut zur Hemmung von Sebumsekretion.

2. Verwendung nach Anspruch 1, wobei die Matrixmetalloproteinase eine zur Gelatinasegruppe gehörende Proteinase ist.

3. Verwendung nach Anspruch 1, wobei der Matrixmetalloproteinaseinhibitor aus der Gruppe von einer aktiven Substanz A der im Folgenden angegebenen Formel,

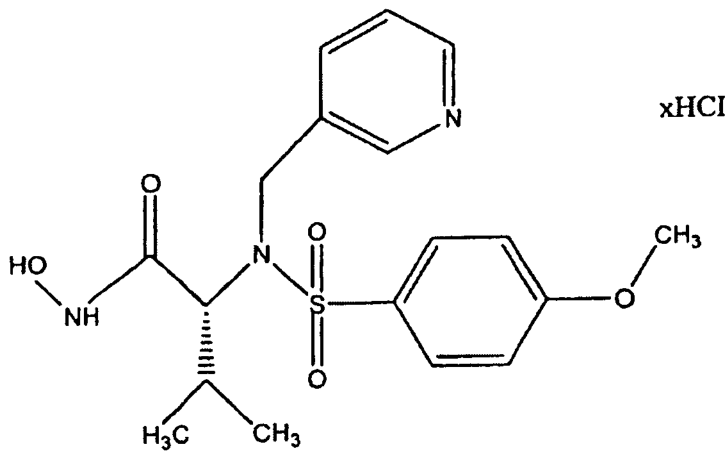

xHCl

einem Extrakt von Patentilla tormentilla S., Curcumin, einem Extrakt von Persea americana Mill., einem Extrakt von Garcinia mangostana L., einem Extrakt von Cocos nucifera L., einem Extrakt von Blumea balsamifera (L) DC. und einem Extrakt von Cinnamomum cassia B1 ausgewählt ist.

**Revendications**

1. Utilisation d'un inhibiteur de métalloprotéinase de la matrice pour la production d'une composition pour application externe sur la peau pour inhiber la sécrétion de sébum.

2. Utilisation selon la revendication 1, dans laquelle la métalloprotéinase de la matrice est une protéinase appartenant au groupe de la gélatinase.

3. Utilisation selon la revendication 1, dans laquelle ledit inhibiteur de métalloprotéinase de la matrice est choisi dans le groupe constitué par la substance active A représentée par la formule suivante :

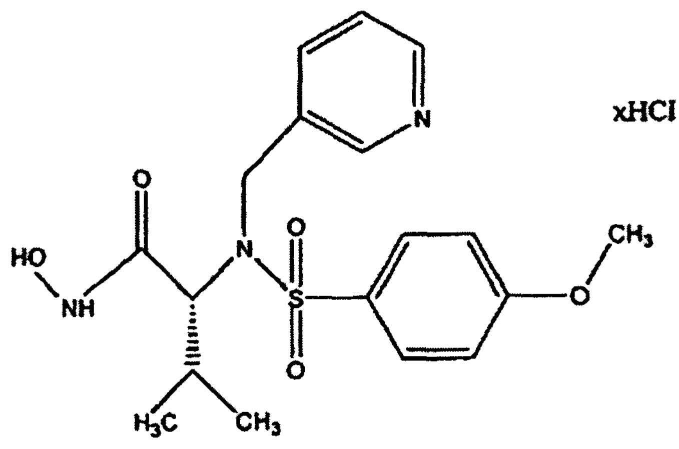

xHCl

un extrait de Patentilla tormentilla S., Curcumine, un extrait de Persea americana Mill., un extrait de Garcinia mangostana L., un extrait de Cocos nucifera L., un extrait de Blumea balsamifera (L) DC., et un extrait de Cinnamomum cassia B1.

Fig.1

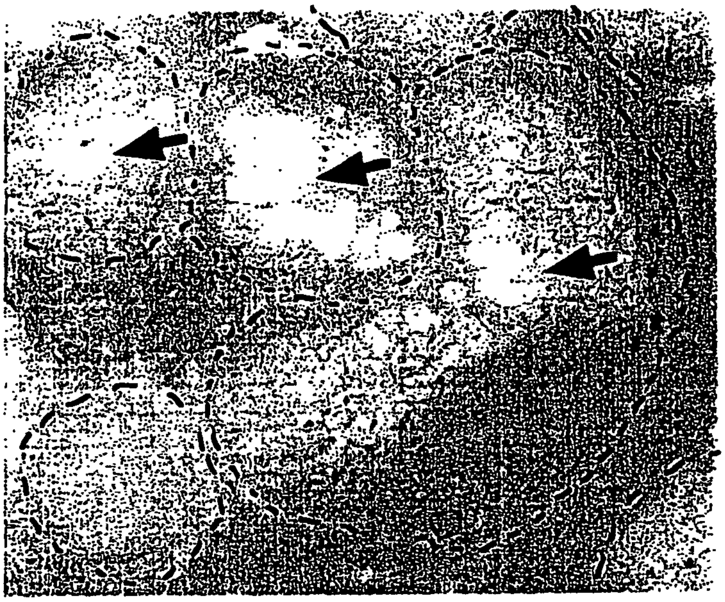

A

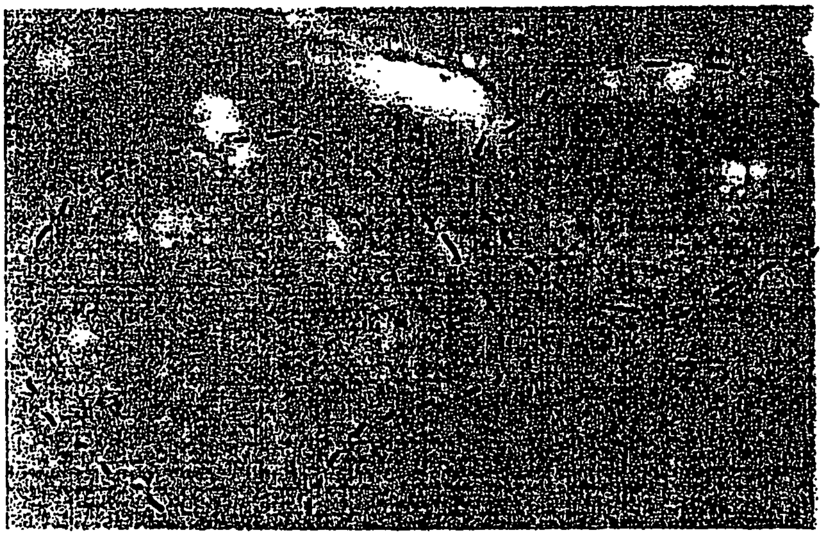

B

Fig. 2

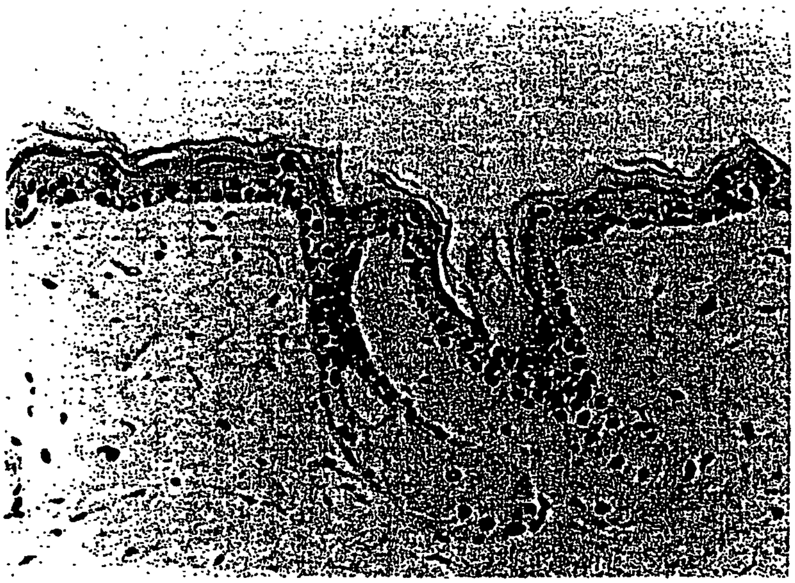

A

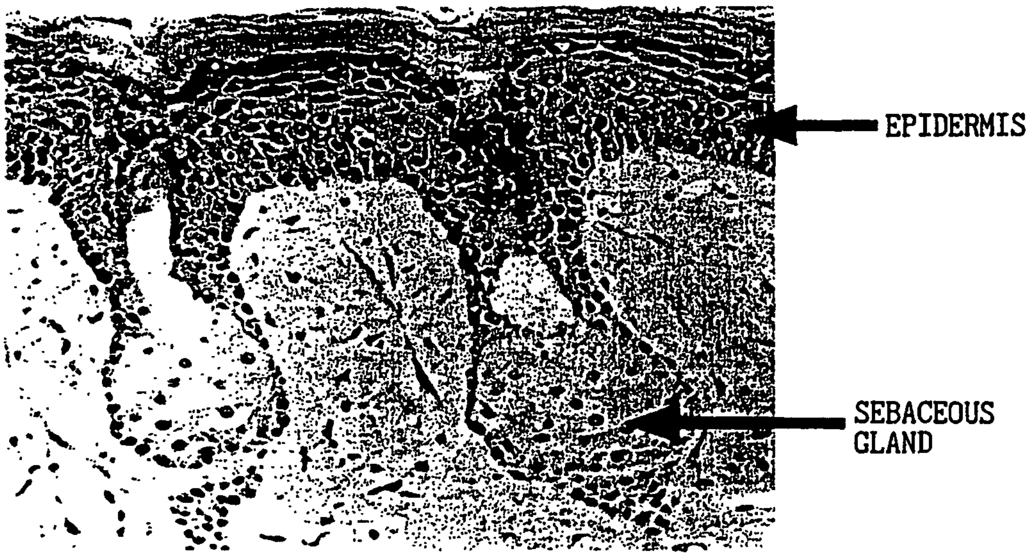


B

Fig.3

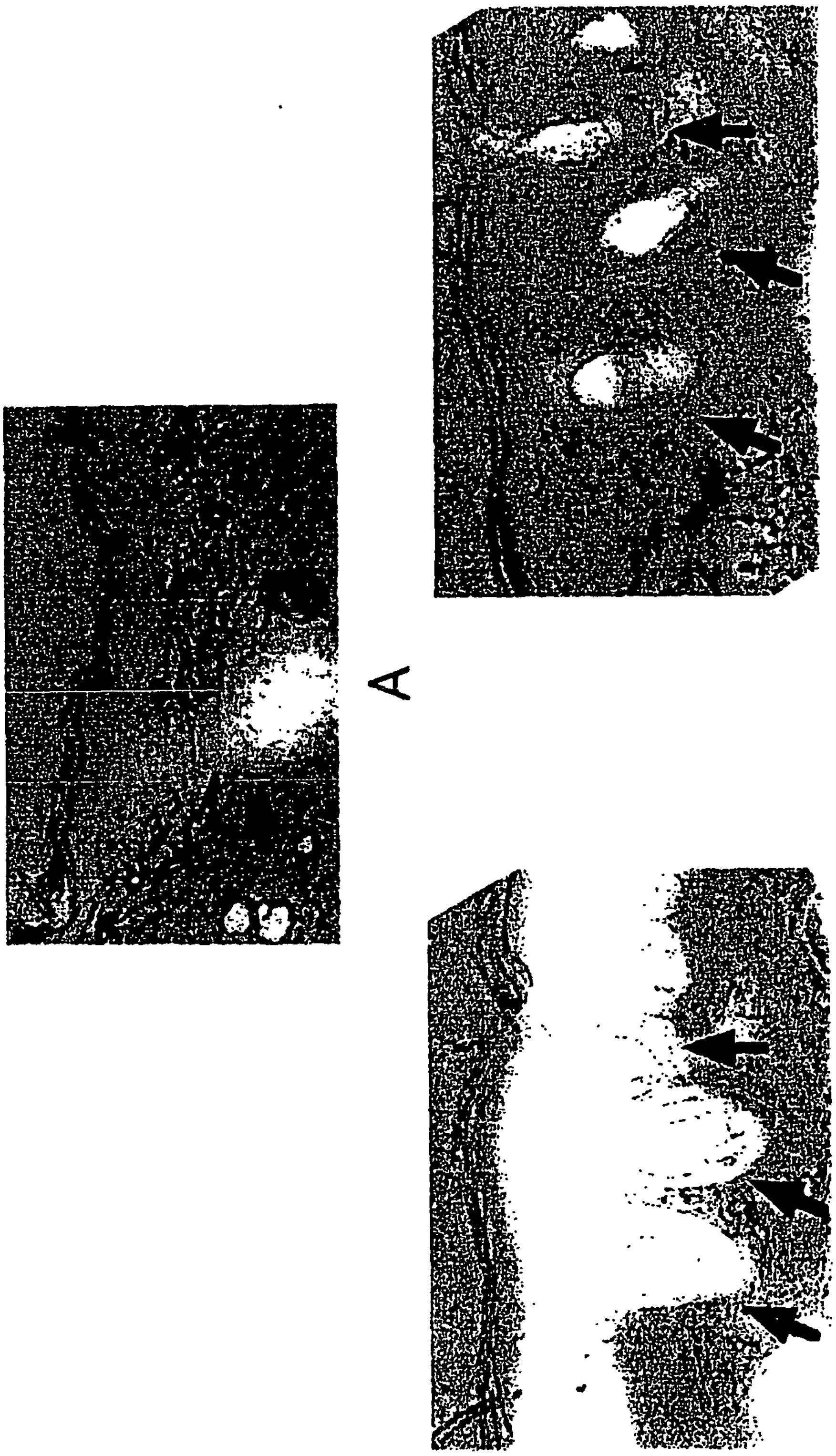

A

B

C

Fig. 4

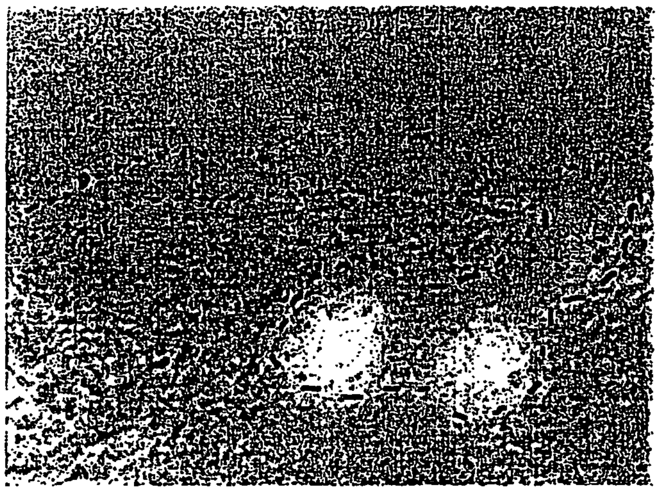

A

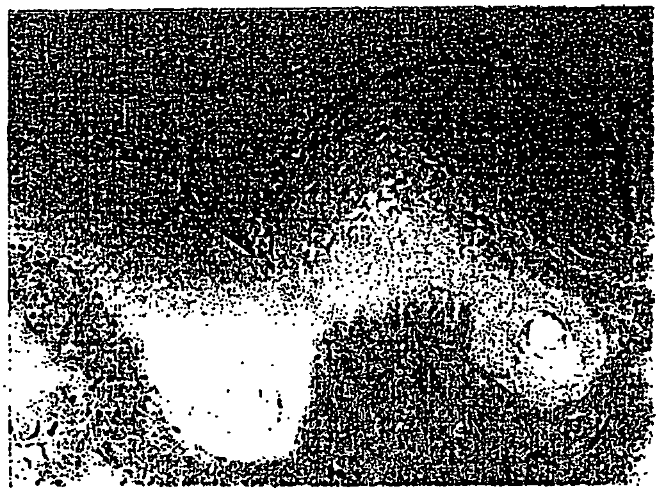

B